(19) Europäisches Patentamt
European Patent Office
Office européen des brevets

(11) **EP 2 698 108 A1**

(12) **EUROPEAN PATENT APPLICATION**

(43) Date of publication:
**19.02.2014 Bulletin 2014/08**

(51) Int Cl.:
***A61B 5/055*** *(2006.01)*

(21) Application number: **12180656.6**

(22) Date of filing: **16.08.2012**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Max-Planck-Gesellschaft zur Förderung**
**der Wissenschaften e.V.**
**80539 München (DE)**

(72) Inventor: **The designation of the inventor has not yet been filed**

(74) Representative: **Vossius & Partner**
**Siebertstrasse 4**
**81675 München (DE)**

(54) **Modification of the Axonal Microstructure in a Mouse Model of Mild Traumatic Brain Injury**

(57) The present invention relates to a method of screening for a compound decreasing axonal injury, comprising the steps of (a) exposing the head of a non-human mammal to a pressure of 12 to 17 psi in a shock tube for a time $t_2$ calculated by the formula

$$t_2 = (m_1/m_2)^{-1/3} \times t_1,$$

wherein $m_2$ is the weight of the brain of the non-human mammal, $m_1$ is 480 mg and $t_1$ is 1.0 to 2.0 msec, (b) administering a test compound to the non-human mammal obtained in (a), and/or (b') administering a test compound to the non-human mammal before step (a), and (c) measuring the fractional anisotropy in the brain of the non-human mammal by diffusion tensor imaging after performing steps (a) and (b), or (a) and (b'), or (a), (b) and (b'), wherein a test compound is a compound decreasing axonal injury if (i) in the case of (b) and/or (b'): the fractional anisotropy measured in step (c) is increased as compared to the fractional anisotropy measured in the brain of a control non-human mammal of the same species which has been exposed to pressure as defined in step (a), and/or (ii) in the case of (b): the fractional anisotropy measured in step (c) is increased as compared to the fractional anisotropy measured in the brain of said non-human mammal after step (a) and before the administration of the test compound in step (b).

**Description**

The present invention relates to a method of screening for a compound decreasing axonal injury, comprising the steps of (a) exposing the head of a non-human mammal to a pressure of 12 to 17 psi in a shock tube for a time $t_2$ calculated by the formula

$$t_2 = (m_1/m_2)^{-1/3} \times t_1,$$

wherein $m_2$ is the weight of the brain of the non-human mammal, $m_1$ is 480 mg and $t_1$ is 1.0 to 2.0 msec, (b) administering a test compound to the non-human mammal obtained in (a), and/or (b') administering a test compound to the non-human mammal before step (a), and (c) measuring the fractional anisotropy in the brain of the non-human mammal by diffusion tensor imaging after performing steps (a) and (b), or (a) and (b'), or (a), (b) and (b'), wherein a test compound is a compound decreasing axonal injury if (i) in the case of (b) and/or (b'): the fractional anisotropy measured in step (c) is increased as compared to the fractional anisotropy measured in the brain of a control non-human mammal of the same species which has been exposed to pressure as defined in step (a), and/or (ii) in the case of (b): the fractional anisotropy measured in step (c) is increased as compared to the fractional anisotropy measured in the brain of said non-human mammal after step (a) and before the administration of the test compound in step (b).

In this specification, a number of documents including patent applications and manufacturer's manuals is cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

Traumatic Brain Injury (TBI) is an important global public health problem. In the United States, an average of 1.4 million TBIs and 50,000 associated deaths occur each year (1). The leading causes are falls and motor vehicle crashes (2). TBI has been recognized as the "signature injury" in the recent war zones of Iraq and Afghanistan (3, 4). Detonation of Improvised Explosive Devices (IEDs) generates very hot, dense, and high pressure gas which forms a shock wave characterized as a Friedlander wave (5, 6, 7). Clinical severity is correlated with the duration of unconsciousness (8). Approximately, 80% of hospital-reported patients suffering from TBI are categorized as mild TBI (9). A similar prevalence applies to the military sector (10).

In view of the above-discussed prevalence of Traumatic Brain Injury there is a need for screening methods which may lead to the identification of new therapeutics for the treatment of Traumatic Brain Injury, in particular mild Traumatic Brain Injury. There is also a need for novel treatments of Traumatic Brain Injury, in particular mild Traumatic Brain Injury and other diseases associated with myelin abnormalities, especially demyelinating diseases such as Multiple Sclerosis. There is furthermore a need for methods for monitoring the regeneration of Traumatic Brain Injury and diseases associated with axonal abnormalities. These needs are addressed by the present invention.

In a first embodiment the present invention relates to a method of screening for a compound decreasing axonal injury, comprising the steps of (a) exposing the head of a non-human mammal to a pressure of 12 to 17 psi in a shock tube for a time $t_2$ calculated by the formula

$$t_2 = (m_1/m_2)^{-1/3} \times t_1,$$

wherein $m_2$ is the weight of the brain of the non-human mammal, $m_1$ is 480 mg and $t_1$ is 1.0 to 2.0 msec, (b) administering a test compound to the non-human mammal obtained in (a), and/or (b') administering a test compound to the non-human mammal before step (a), and (c) measuring the fractional anisotropy in the brain of the non-human mammal by diffusion tensor imaging after performing steps (a) and (b), or (a) and (b'), or (a), (b) and (b'), wherein a test compound is a compound decreasing axonal injury if (i) in the case of (b) and/or (b'): the fractional anisotropy measured in step (c) is increased as compared to the fractional anisotropy measured in the brain of a control non-human mammal of the same species which has been exposed to pressure as defined in step (a), and/or (ii) in the case of (b): the fractional anisotropy measured in step (c) is increased as compared to the fractional anisotropy measured in the brain of said non-human mammal after step (a) and before the administration of the test compound in step (b).

Axonal injury as used herein designates any damage or impairment (for example the disruption) of neuronal axons (including the myelin microstructure) in the brain. It is preferred that the axonal injuries are those which are associated with mild Traumatic Brain Injury (TBI) (9).

In accordance with the present invention, a TBI is a traumatically induced structural injury and/or physiological disruption of brain function as a result of an external force that is indicated by new onset or worsening of at least one of the following clinical signs, immediately following the event: (i) any period of loss of or a decreased level of consciousness; (ii) any loss of memory for events immediately after the injury; (iii) any alteration in mental state at the time of the injury (confusion, disorientation, slowed thinking, etc.); and (iv) neurological deficits (weakness, loss of balance, change in vision, praxis, paresis/plegia, sensory loss, aphasia, etc.) that may or may not be transient (see Department of Defense and Department of Veterans Affairs (2008). "Traumatic Brain Injury Task Force". http://www.cdc.gov/nchs/data/icd9/Sep08TBI.pdf). TBI causes structural damage and/or produces more subtle damage of the brain, the latter being manifested by altered brain function without detectable structural damage.

External forces may include any of the following events: the brain undergoing an acceleration/deceleration movement without direct external trauma to the head, or forces generated from events such as a blast or explosion. It is preferred in accordance with the invention that the external forces are usually generated from events such as a blast or explosion.

TBI varies in severity, traditionally described as mild, moderate and severe. These categories are based on the Glasgow Coma Score (GCS). For mild, moderate and severe TBI, the GCS is 13-15, 9-13 and 8 or less, respectively. Important are measures of length of unconsciousness or post traumatic amnesia, which are well-established. Classification systems typically differentiate TBI on the basis of loss of consciousness (LOC), altered consciousness (AOC), and post-traumatic amnesia (PTA). In accordance with the present invention, TBI is preferably mild TBI and more preferably blast-induced mild TBI.

A compound decreasing axonal injury in accordance with the present invention may ameliorate an axonal injury, if the compound is administered before and/or after the blast, or prevent axonal injury, if the compound is at least administered before the blast.

It has to be understood that the test compound is not administered to the control non-human mammal of the same species in accordance with (i). Notwithstanding, a negative control compound, e.g. a compound known to not decrease axonal injury, may be administered to the control non-human mammal.

The pressure of 12 to 17 psi (pound-force per square inch) is, with increasing preference, 13 to 17 psi, 14 to 17 psi and 15 to 17psi. 1 psi corresponds to about 69mbar.

$m_1$ is 480 mg. 480mg is the average brain mass of a male C57BL/6N mice used in the examples herein below.

The time $t_1$ is, with increasing preference, 1.2 to 1.8 msec, 1.3 to 1.6 msec, and 1.4 msec.

The formula indicates the Holbourn's scaling law which is described in greater detail in Example 1, material and methods, herein below. Translation of the exposure times to model mammals other than mouse such as rats, or other non-human primates described herein can be done by the skilled person with Holbourn's scaling laws. A 1.4 msec exposure to a mouse brain is approximately comparable to an exposure of 20 msec to a human brain. Such an exposure can be experienced when a human is exposed to large explosions at a distance. Performing step (a) of the method described herein above is believed to result in a blast-induced mild TBI in the non-human mammal.

A shock tube is an instrument used to replicate very hot, dense, and high pressure gas waves from Improvised Explosive Devices (IBD's) in order to simulate actual explosions and their effects, usually on a smaller scale. As is shown in the examples employing mice below, an aluminum 6 ft long and 2 inches wide tube consisting of a Driver Section to be filled with Helium gas, an Expansion Chamber separated from the Driver Section by a Mylar Diaphragm that bursts at a defined pressure was used. A Mouse Chamber was added at the end of the tube. It holds the mouse in a vertical position to the tube. For each blast, the pressure is measured at the exit surface of the tube and recorded with a digital pressure gauge. More recently, shock tubes have been used in biomedical research to study how biological specimens are affected by blast waves. The advantage of shock tubes is that the main parameters can be tailored to defined research aims and be easily reproduced. An important feature is the quick rise of the high-pressure curve and its rapid return to baseline with a short negative phase and short exposure time. As illustrated in Example 1, materials and methods, using, for example, helium helps to achieve these objectives. The exposure times in small animal models, for example a mouse, should be in the range of a couple of msec to be realistically comparable to conditions affecting humans. On the basis of these considerations, the shock tube is currently also employed in other small animal models of mild TBI (21, 22, 23, 24, 25). The shock tube used in the method of the invention is preferably a Helium-driven shock tube.

In the examples appended below, a shock tube that generates a high Helium pressure wave similar to the pressure wave from Improvised Explosive Devices (IEDs) in war zones was used. It was an objective of the present invention to determine (i) whether the blast generated by the shock tube modified fractional anisotropy (FA), and (ii) whether behavior and FA changes were significantly correlated, in such way that greater freezing was linked to lower FA and vice versa. In this context, direct effects of DFC or TFC on FA and their potential therapeutic action had to be considered.

A test compound may be any compound which potentially decreases axonal injury. Test compounds include but are not limited to a virus, protein, peptide, amino acid, lipid, small molecule, carbohydrate, nucleic acid (e.g. an siRNA, an shRNA, an miRNA, an siRNA, an shRNA, an miRNA), nucleotide, drug, antibody, aptamer/spiegelmer, prodrug, ribozyme or combinations thereof.

The term "protein" as used herein refers to any high molecular mass compound consisting of one or more linear chains of the 20 amino acids of the genetic code joined by peptide bonds, occuring in living systems. The amino acids in the chains may be naturally modified by e,g, glycosylation, acetylation, phosphorylation and similar modifications which are well known in the art.

The term "protein" as used herein includes proteins soluble in aqueous solvents, like enzymes such as kinases, proteases, phosphatases, oxidases and reductases as well as non-enzyme soluble proteins, for example soluble proteins comprising immunoglobulin domains such as soluble antibodies. Membrane proteins, comprising peripheral membrane proteins and integral membrane proteins are also included. Examples of membrane proteins include transporters, pores and receptors such as growth factor receptors, G-protein coupled receptors (GPCR) and receptors with seven, but also with less or more than seven transmembrane helices, or beta sheets. The term "protein" refers to single-domain proteins as well as to multi-domain proteins and to single chain as well as multiple chain proteins. Further embraced are embodiments, wherein the protein functions as part of a metabolic pathway and/or a signal transduction pathway. The protein may consist of or comprise protein domains known to function in signal transduction and/or known to be involved in protein-protein interaction. Examples for such domains are Ankyrin repeats; arm, Bcl-homology, Bromo, CARD, CH, Chr, C1, C2, DD, DED, DH, EFh, ENTH, F-box, FHA, FYVE, GEL, GYF, hect, LIM, MH2, PDZ, PB1, PH, PTB, PX, RGS, RING, SAM, SC, SH2, SH3, SOCS, START, TIR, TPR, TRAF, tsnare, Tubby, UBA, VHS, W, WW, and 14-3-3 domains. Further information about these and other protein domains is available from the databases InterPro (http://www.ebi.ac.uk/interpro/, Mulder et al., 2003), Pfam (http://www.sanger.ac.uk/Software/Pfam/, Bateman et al., 2004) and SMART (http://smart.embl-heidelberg.de/, Letunic et al., 2004). Peptides are short polymers of amino acid monomers linked by peptide bonds, preferably comprising less than 50 amino acids, more preferably less than 25 amino acids and most preferably less than 10 amino acids.

A carbohydrate is an organic compound that consists only of carbon, hydrogen, and oxygen, usually with the empirical formula $C_m(H_2O)_n$.

A drug may be any substance that, when absorbed into the body of a living organism, alters normal bodily function. In pharmacology, a drug is a chemical substance used in the treatment, cure, prevention, or diagnosis of disease or used to otherwise enhance physical or mental well-being.

A "prodrug" is a compound that is generally not biologically and/or pharmacologically active. After administration, the prodrug is activated, typically *in vivo* by enzymatic or hydrolytic cleavage and converted to a biologically and/or pharmacologically active compound which has, the intended medical effect. Prodrugs are typically formed by chemical modification of biologically and/or pharmacologically compound. Conventional procedures for the selection and preparation of suitable prodrug derivatives are described, for example, in Design of Prodrugs, ed. H. Bundgaard, Elsevier, 1985.

A "small molecule" according to the present invention may be, for example, an organic molecule. Organic molecules relate or belong to the class of chemical compounds having a carbon basis, the carbon atoms linked together by carbon-carbon bonds. The original definition of the term organic related to the source of chemical compounds, with organic compounds being those carbon-containing compounds obtained from plant or animal or microbial sources, whereas inorganic compounds were obtained from mineral sources. Organic compounds can be natural or synthetic. Alternatively, the "small molecule" in accordance with the present invention may be an inorganic compound. Inorganic compounds are derived from mineral sources and include all compounds without carbon atoms (except carbon dioxide, carbon monoxide and carbonates). Preferably, the small molecule has a molecular weight of less than about 2000 amu, or less than about 1000 amu such as less than about 500 amu, and even more preferably less than about 250 amu. The size of a small molecule can be determined by methods well-known in the art, e.g., mass spectrometry. The small molecules may be designed, for example, based on the crystal structure of a target molecule, where sites presumably responsible for the biological activity, can be identified and verified in *in vivo* assays such as *in vivo* high-throughput screening (HTS) assays. Such small molecules may be particularly suitable as inhibitors by blocking specific bindings sites of said target molecule.

The term "antibody" as used in accordance with the present invention comprises polyclonal and monoclonal antibodies, as well as derivatives or fragments thereof, which still retain binding specificity. Antibody fragments or derivatives comprise, inter alia, Fab or Fab' fragments as well as Fd, F(ab')$_2$, Fv or scFv fragments; see for example Harlow and Lane "Antibodies, A Laboratory Manual", Cold Spring Harbor Laboratory Press, 1988 and Harlow and Lane "Using Antibodies: A Laboratory Manual" Cold Spring Harbor Laboratory Press, 1999. The term "antibody" also includes embodiments such as chimeric (human constant domain, non-human variable domain), single chain and humanized (human antibody with the exception of non-human CDRs) antibodies.

Various techniques for the production of antibodies are well known in the art and described, e.g. in Harlow and Lane (1988) and (1999), loc. cit. In addition, the antibodies can be produced as peptidomimetics. Further, techniques described for the production of single chain antibodies (see, inter alia, US Patent 4,946,778) can be adapted to produce single chain antibodies specific for the target of this invention. Also, transgenic animals or plants (see, e.g., US patent 6,080,560) may be used to express (humanized) antibodies specific for the target of this invention. Most preferably, the antibody is a monoclonal antibody, such as a human or humanized antibody. For the preparation of monoclonal antibodies, any technique which provides antibodies produced by continuous cell line cultures can be used. Examples for such techniques are described, e.g. in Harlow and Lane (1988) and (1999), loc. cit. and include the hybridoma technique originally developed by Köhler and Milstein Nature 256 (1975), 495-497, the trioma technique, the human B-cell hybridoma technique (Kozbor, Immunology Today 4 (1983), 72) and the EBV-hybridoma technique to produce human monoclonal antibodies (Cole et al., Monoclonal Antibodies and Cancer Therapy, Alan R. Liss, Inc. (1985), 77-96). Surface plasmon resonance as employed in the BIAcore system can be used to increase the efficiency of phage antibodies which bind to an epitope of the target protein (Schier, Human Antibodies Hybridomas 7 (1996), 97-105; Malmborg, J. Immunol. Methods 183 (1995), 7-13). It is also envisaged in the context of this invention that the term "antibody" comprises antibody constructs which may be expressed in cells, e.g. antibody constructs which may be transfected and/or transduced via, inter alia, viruses or plasmid vectors.

Aptamers are nucleic acid or peptide molecules that bind a specific target molecule. More specifically, aptamers can be classified as nucleic acid aptamers, such as DNA or RNA aptamers, or peptide aptamers. Whereas the former normally consist of (usually short) strands of oligonucleotides, the latter preferably consist of a short variable peptide domain, attached at both ends to a protein scaffold. Whereas nucleic acid aptamers are nucleic acid molecules that are in the natural D-conformation, the corresponding nucleic acid molecules that are in the L-conformation are referred to in the art and herein as spiegelmers.

Aptamers, as well as spiegelmers, are usually created by selecting them from a large random sequence pool, but natural aptamers also exist in riboswitches. Aptamers and spiegelmers can be used as macromolecular drugs. They can be combined with ribozymes to self-cleave in the presence of their target molecule. These compound molecules have additional research, industrial and clinical applications (Osborne et. al. (1997), Current Opinion in Chemical Biology, 1:5-9; Stull & Szoka (1995), Pharmaceutical Research, 12, 4:465-483).

In accordance with the present invention, the term "small interfering RNA (siRNA)", also known as short interfering RNA or silencing RNA, refers to a class of 18 to 30, preferably 19 to 25, most preferred 21 to 23 or even more preferably 21 nucleotide-long double-stranded RNA molecules that play a variety of roles in biology. Most notably, siRNA is involved in the RNA interference (RNAi) pathway where the siRNA interferes with the expression of a specific gene. In addition to their role in the RNAi pathway, siRNAs also act in RNAi-related pathways, e.g. as an antiviral mechanism or in shaping the chromatin structure of a genome.

siRNAs naturally found in nature have a well defined structure: a short double-strand of RNA (dsRNA) with 2-nt 3' overhangs on either end. Each strand has a 5' phosphate group and a 3' hydroxyl (-OH) group. This structure is the result of processing by dicer, an enzyme that converts either long dsRNAs or small hairpin RNAs into siRNAs. siRNAs can also be exogenously (artificially) introduced into cells to bring about the specific knockdown of a gene of interest. Essentially any gene of which the sequence is known can thus be targeted based on sequence complementarity with an appropriately tailored siRNA. The double-stranded RNA molecule or a metabolic processing product thereof is capable of mediating target-specific nucleic acid modifications, particularly RNA interference and/or DNA methylation. Exogenously introduced siRNAs may be devoid of overhangs at their 3' and 5' ends, however, it is preferred that at least one RNA strand has a 5'- and/or 3'-overhang. Preferably, one end of the double-strand has a 3'-overhang from 1-5 nucleotides, more preferably from 1-3 nucleotides and most preferably 2 nucleotides. The other end may be blunt-ended or has up to 6 nucleotides 3'-overhang. In general, any RNA molecule suitable to act as siRNA is envisioned in the present invention. The most efficient silencing was so far obtained with siRNA duplexes composed of 21-nt sense and 21-nt antisense strands, paired in a manner to have 2-nt 3' overhangs on either end. The sequence of the 2-nt 3' overhang makes a small contribution to the specificity of target recognition restricted to the unpaired nucleotide adjacent to the first base pair (Elbashir et al. 2001). 2'-deoxynucleotides in the 3' overhangs are as efficient as ribonucleotides, but are often cheaper to synthesize and probably more nuclease resistant. Delivery of siRNA may be accomplished using any of the methods known in the art, for example by combining the siRNA with saline and administering the combination intravenously or intranasally or by formulating siRNA in glucose (such as for example 5% glucose) or cationic lipids and polymers can be used for siRNA delivery in vivo through systemic routes either intravenously (IV) or intraperitoneally (IP) (Fougerolles et al. (2008), Current Opinion in Pharmacology, 8:280-285; Lu et al. (2008), Methods in Molecular Biology, vol. 437: Drug Delivery Systems - Chapter 3: Delivering Small Interfering RNA for Novel Therapeutics).

A short hairpin RNA (shRNA) is a sequence of RNA that makes a tight hairpin turn that can be used to typically silence gene expression via RNA interference. shRNA can for example use a vector introduced into cells, in which case preferably the U6 promoter is utilized to ensure that the shRNA is always expressed. This vector is usually passed on to daughter cells, allowing the gene silencing to be inherited. The shRNA hairpin structure is cleaved by the cellular machinery into siRNA, which is then bound to the RNA-induced silencing complex (RISC). This complex binds to and cleaves mRNAs which match the siRNA that is bound to it.

Preferably, si/shRNAs to be used in the present invention are chemically synthesized using conventional methods that, for example, appropriately protected ribonucleoside phosphoramidites and a conventional DNA/RNA synthesizer. Suppliers of RNA synthesis reagents are Proligo (Hamburg, Germany), Dharmacon Research (Lafayette, CO, USA), Pierce Chemical (part of Perbio Science, Rockford, IL, USA), Glen Research (Sterling, VA, USA), ChemGenes (Ashland, MA, USA), and Cruachem (Glasgow, UK). Most conveniently, siRNAs or shRNAs are obtained from commercial RNA oligo synthesis suppliers, which sell RNA-synthesis products of different quality and costs. In general, the RNAs applicable in the present invention are conventionally synthesized and are readily provided in a quality suitable for RNAi.

Further molecules effecting RNAi include, for example, microRNAs (miRNA). Said RNA species are single-stranded RNA molecules which, as endogenous RNA molecules, regulate gene expression. Binding to a complementary mRNA transcript triggers the degradation of said mRNA transcript through a process similar to RNA interference. Accordingly, miRNA may be employed as an inhibitor of the calcium-activated chloride channel of the present invention.

A ribozyme (from ribonucleic acid enzyme, also called RNA enzyme or catalytic RNA) is an RNA molecule that catalyzes a chemical reaction. Many natural ribozymes catalyze either their own cleavage or the cleavage of other RNAs, but they have also been found to catalyze the aminotransferase activity of the ribosome. Non-limiting examples of well-characterized small self-cleaving RNAs are the hammerhead, hairpin, hepatitis delta virus, and *in vitro*-selected lead-dependent ribozymes, whereas the group I intron is an example for larger ribozymes. The principle of catalytic self-cleavage has become well established in the last 10 years. The hammerhead ribozymes are characterized best among the RNA molecules with ribozyme activity. Since it was shown that hammerhead structures can be integrated into heterologous RNA sequences and that ribozyme activity can thereby be transferred to these molecules, it appears that catalytic antisense sequences for almost any target sequence can be created, provided the target sequence contains a potential matching cleavage site. The basic principle of constructing hammerhead ribozymes is as follows: An interesting region of the RNA, which contains the GUC (or CUC) triplet, is selected. Two oligonucleotide strands, each usually with 6 to 8 nucleotides, are taken and the catalytic hammerhead sequence is inserted between them. Molecules of this type were synthesized for numerous target sequences. They showed catalytic activity in vitro and in some cases also in vivo. The best results are usually obtained with short ribozymes and target sequences.

A recent development, is the combination of an aptamer recognizing a small compound with a hammerhead ribozyme. The conformational change induced in the aptamer upon binding the target molecule is supposed to regulate the catalytic function of the ribozyme.

The term "antisense nucleic acid molecule" is well known in the art and refers to a nucleic acid which is complementary to a target nucleic acid, i.e. a nucleic acid encoding a target protein. An antisense molecule in accordance with the invention is capable of interacting with the target nucleic acid, more specifically it is capable of hybridizing with the target nucleic acid. Due to the formation of the hybrid, transcription of the target gene(s) and/or translation of the target mRNA is reduced or blocked. Standard methods relating to antisense technology have been described (see, e.g., Melani et al., Cancer Res. (1991) 51:2897-2901).

Also encompassed herein are modified versions of these inhibitory compounds.

The term "modified versions of these inhibitory compounds" in accordance with the present invention refers to versions of the compounds that are modified to achieve i) modified spectrum of activity, organ specificity, and/or ii) improved potency, and/or iii) decreased toxicity (improved therapeutic index), and/or iv) decreased side effects, and/or v) modified onset of therapeutic action, duration of effect, and/or vi) modified pharmacokinetic parameters (resorption, distribution, metabolism and excretion), and/or vii) modified physico-chemical parameters (solubility, hygroscopicity, color, taste, odor, stability, state), and/or viii) improved general specificity, organ/tissue specificity, and/or ix) optimised application form and route by, for example, (a) esterification of carboxyl groups, or (b) esterification of hydroxyl groups with carboxylic acids, or (c) esterification of hydroxyl groups to, e.g. phosphates, pyrophosphates or sulfates or hemisuccinates, or (d) formation of pharmaceutically acceptable salts, or (e) formation of pharmaceutically acceptable complexes, or (f) synthesis of pharmacologically active polymers, or (g) introduction of hydrophilic moieties, or (h) introduction/exchange of substituents on aromates or side chains, change of substituent pattern, or (i) modification by introduction of isosteric or bioisosteric moieties, or (j) synthesis of homologous compounds, or (k) introduction of branched side chains, or (k) conversion of alkyl substituents to cyclic analogues, or (l) derivatisation of hydroxyl groups to ketales, acetales, or (m) N-acetylation to amides, phenylcarbamates, or (n) synthesis of Mannich bases, imines, or (o) transformation of ketones or aldehydes to Schiff's bases, oximes, acetales, ketales, enolesters, oxazolidines, thiazolidines; or combinations thereof.

The various steps recited above are generally known in the art. They include or rely on quantitative structure-action relationship (QSAR) analyses (Kubinyi, "Hausch-Analysis and Related Approaches", VCH Verlag, Weinheim, 1992), combinatorial biochemistry, classical chemistry and others (see, for example, Holzgrabe and Bechtold, Deutsche Apotheker Zeitung 140(8), 813-823, 2000).

Diffusion tensor imaging (DTI) is a magnetic resonance imaging (MRI) technique that enables the measurement of the restricted diffusion of water in tissue in order to produce neural tract images instead of using this data solely for the purpose of assigning contrast or colors to pixels in a cross sectional image. DTI is used in the art when a tissue - such as the neural axons of white matter in the brain - has an internal fibrous structure analogous to the anisotropy of some crystals. From the diffusion tensor, diffusion anisotropy measures, such as the fractional anisotropy (FA), can be computed by methods well-known to the skilled person in the field of MRI (e.g., Le Bihan, D; E. Breton (1985), C R Acad Sci (Paris) 301 (15): 1109-1112 or Hagmann et al., Radio Graphics, Oct 2006).

Fractional anisotropy (FA) is a scalar value between 0 and 1 that describes the degree of anisotropy of a diffusion process. A value of 0 means that diffusion is isotropic, i.e. it is unrestricted (or equally restricted) in all directions. A value of 1 means that diffusion occurs only along one axis and is fully restricted along all other directions. FA is a measure often used in diffusion imaging, like DTI, where it is believed to reflect fiber density, axonal diameter, and myelination in white matter. The FA is an extension of the concept of eccentricity of conic sections in 3 dimensions, normalized to the unit range. The determination of FA is, for example, described in Westin et al., Geometrical diffusion measures for MRI from tensor basis analysis, In ISMRM '97, Vancouver Canada, 1997; 1742 or Westin et al., Processing and visualization of diffusion tensor MRI, Medical Image Analysis 2002; 6(2): 93-108.

As it can be taken from the examples herein below, evidence for axonal injuries can be provided by low FA values determined with DTI. An increase of FA is indicative for myelin synthesis and rebuilding of myelin organization. Hence, a decrease of FA is indicative for myelin abnormalities, which are further defined herein below.

The non-human mammal may be, for example, a swine, rat, dog, cat, sheep, goat, cattle, monkey, or ape.

It has to be understood that either of steps (b), (b') and (c), or only step (c), may be repeated. Repeating steps (b), (b') and (c) may allow for testing more than one test compound in the same non-human mammal. Repeating step (c) may allow for monitoring the effects of a test compound over time. Furthermore, it is also possible to administer more than one test compound at the same time in step (b) and/or (b'). In case steps (b) and (b') are performed, the administered test compounds may be the same or different.

In accordance with the present invention, a test compound is considered to be a compound decreasing axonal injury when the fractional anisotropy measured in step (c) is increased as compared to either a different control animal of the same species (i.e. option (i)) or the same animal used in the screening method but prior to application of the test compound (i.e. option (ii)).

It follows that option (i), i.e. a comparison to a different animal of the same species, is applicable irrespective of whether the test compound is administered prior to step (a), subsequently to step (a), or both. Accordingly, option (i) is applicable in those cases where the method comprises either step (b), or step (b') or both steps (b) and (b'). Option (ii), on the other hand, is only applicable when the test compound is administered subsequently to step (a), i.e. where the method comprises solely step (b) but not step (b').

Once a compound decreasing axonal injury has been identified by the method of the invention, said compound may be further administered to a non-human mammal, the FA may be measured in said non-human mammal and compared to the FA measured in a naïve non-human mammal of the same species, which did not receive the compound decreasing axonal injury, thereby determining whether the compound decreasing axonal injury has an effect on FA in naïve non-human mammals (i.e. unblasted non-human mammals).

As it can be taken from the examples, the invention has been exemplified by using a mouse as non-human mammal.

Accordingly in a preferred embodiment of the method of the invention described-above, the non-human mammal is a mouse.

As is shown in the appended examples, the behavioral changes in fear conditioning and alterations in diffusion tensor imaging of the corpus callosum were observed after mice received a 1.4 msec blast of 16 psi helium in the study of mild TBI. It was observed that the blast increased contextual freezing to the training context and to a novel context in the cue test. Also decreased normalized freezing in response to exposure to the cue in the novel context has been found. The animals lost the ability to respond specifically to the contextual stimuli of the conditioning stimulus (CS) as indicated by the generalized preCue freezing. The response to the auditory stimulus was weakened as demonstrated by the reduction of the normalized freezing to the cue stimulus. These two responses have been typically ascribed to PTSD behavior (26). The increased preCue freezing is suggestive of generalization and was probably induced by anxiety generated by the blast process. The blast-induced increase of contextual fear is interpreted to be in agreement with Kaouane et al (26) and to reflect an expression of PTSD-like behavior, possibly on the basis of hyperarousal. The regression performed analysis showed a significant correlation between the FA in the corpus callosum and increase of contextual fear to the training context. This response may also contain contextual components experienced during the blast process.

It was expected that auditory trace fear conditioning, a model of declarative memory, would be more impaired because of its complex nature and the need for intact forebrain neural networks than the less complex nondeclarative memory modeled with auditory delay fear conditioning. However, that was not the case on Day 4 after the blast as indicated by ANOVA statistics of the observed behavior; see Examples herein below. Based on ANOVA, both memory forms behaviorally responded similarly to the blast wave at this time window. The results of the behavior experiments shown below also suggest that the neural network concept may not apply to the shock tube model three or four days after blast. The blast-induced behavior changes are probably triggered by other yet unknown fast mechanisms.

In a further preferred embodiment of the method of the invention described-above, the shock tube generates a helium-driven pressure wave.

The use of helium may further help to standardize conditions in step (a) of the method described-above.

In another preferred embodiment of the method of the invention described-above, step (c) is performed *ex vivo* or *in vivo.*

The DTI experiments described below targeted at FA changes as measures of microstructure changes were performed *ex vivo,* since direct comparison between *in vivo* and fixed rodent and primate brains have demonstrated the maintenance of diffusion anisotropy after tissue fixation (27, 28). Notwithstanding the FA experiments may also be done *in vivo* (see, for example, Le Bihan, D; E. Breton (1985), C R Acad Sci (Paris) 301 (15): 1109-1112 or Hagmann et al., " RadioGraphics. Oct 2006).

It is also preferred in accordance with the method of the invention that measuring the fractional anisotropy in the brain comprises measuring the fractional anisotropy in the white matter. It is particularly preferred that measuring the fractional anisotropy in the white matter comprises measuring the fractional anisotropy in the corpus callosum.

As discussed herein above, DTI is particularly advantage to image neural axons of white matter in the brain due to the texture of the white matter. White matter is one of the two components of the central nervous system and consists mostly of glial cells and myelinated axons that transmit signals from one region of the cerebrum to another and between the cerebrum and lower brain centers. The corpus callosum is a wide, flat bundle of neural fibers beneath the cortex in the eutherian brain at the longitudinal fissure. It connects the left and right cerebral hemispheres and facilitates interhemispheric communication. It is the largest white matter structure in the human brain, consisting of 200-250 million contralateral axonal projections.

In a more preferred embodiment of the method of the invention, wherein the non-human mammal is a mouse, the mouse is exposed to $16.02 \pm 0.6$ psi for a time of 1.4 msec in step (a).

In another preferred embodiment of the method of the invention described-above, the control non-human mammal of the same species is of the same race.

In biology, a species is one of the basic units of biological classification and a taxonomic rank. A species is often defined in the art as a group of organisms capable of interbreeding and producing fertile offspring. In biology, races are distinct, genetically divergent populations within the same species with relatively small morphological and genetic differences. These populations can be described as ecological races, i.e. if they arise from adaptation to different local habitats, or geographic races, i.e. when they are geographically isolated. In this regard is preferred that the control non-human mammal of the same race also has the same gender as the test non-human mammal.

In a second embodiment the present invention relates to an implicit memory task for use in the treatment of myelin abnormalities in a subject.

Also encompassed by the present invention is a method of treating myelin abnormalities in a subject comprising performing an implicit memory task with said subject.

Implicit memory is a type of memory in which previous experiences aid in the performance of a task without conscious awareness of these previous experiences. Implicit forms of memory do not require conscious executive control. A non-limiting example is remembering how to brush your teeth. A person usually does not need to think about how to implement brushing of teeth. Implicit memory has to be held distinct from explicit memory. An example of an explicit memory task would be reciting everything a person can remember after reading a book. By contrast, implicit memory tasks are for example automatic responses, drawn out or elicited by a situation, like clapping after a performance. It is usually believed that a mouse has no consciousness. The differentiation between conscious and non-conscious responses therefore has to be differently approached. Auditory trace fear conditioning is accepted as an animal model of declarative or conscious memory because of its complexity. It requires the simultaneous activation of hippocampal, prefrontal and amygdalar functions. In contrast, auditory delay fear conditioning can serve as non-declarative or implicit memory test.

The subject is preferably a mammal, more preferably a primate and most preferably a human.

Myelin abnormalities comprise any disturbance of myelin maintenance and turnover and in particular the myelin abnormalities associated with TBI, more preferably mild TBI and most preferably blast-induced mild TBI. Myelin is a dielectric (electrically insulating) material that forms a layer, the myelin sheath, usually around the axon of a neuron. It is preferred that myelin abnormalities are characterized by a loss of myelin. Myelin abnormalities comprise demyelination and dysmyelination.

It appears from the results of the examples described herein below that auditory delay fear conditioning (DFC) or auditory trace fear conditioning (TFC) training modify axonal processes as indicated by FA. Modification of FA values by the behavior paradigm, especially DFC, shows the significant plasticity of the brain. The increase of FA in the non-blasted mice may at least in part be induced by stimulation of myelin synthesis (29). Similarly, FA is enhanced by working memory training (30) or learning juggling in humans (31). Therefore, the DTI results could partially be explained by the greater efficiency of DFC compared with TFC to reverse axonal deficiencies. The observation of the DFC effects is encouraging from a therapeutic standpoint since it appears that implicit memory tasks like non-declarative DFC used in the examples herein below on blasted mice reverses the blast-induced FA changes, which changes reflect myelin abnormalities, as evidenced by measuring FA with DTI.

The definitions of terms in the context of the first embodiment of the invention apply mutatis mutandis to the second embodiment of the invention.

In a preferred embodiment of the implicit memory task for the use as defined-above, the myelin abnormalities are demyelination and/or dysmyelination.

Demyelination is the loss of the myelin sheath insulating the nerves, and is the hallmark of some neurodegenerative autoimmune diseases, including the non-limiting examples multiple sclerosis, neuromyelitis optica, acute disseminated encephalomyelitis, transverse myelitis, chronic inflammatory demyelinating polyneuropathy, Guillain-Barré Syndrome, central pontine myelinosis, Marchiafava-Bignami disease, inherited demyelinating diseases such as Leukodystrophy, and Charcot Marie Tooth.

Dysmyelination is characterized by a defective structure and function of myelin sheaths; unlike demyelination, it does not produce lesions. Such defective sheaths often arise from genetic mutations affecting the biosynthesis and formation of myelin. Human diseases where dysmyelination has been implicated include leukodystrophies (e.g. Pelizaeus-Merzbacher disease, Canavan disease, phenylketonuria) and schizophrenia.

In a preferred embodiment of the implicit memory task for the use as defined-above, the subject has a traumatic brain injury, blast-induced mild traumatic brain injury, multiple sclerosis, neuromyelitis optica, acute disseminated encephalomyelitis, transverse myelitis, chronic inflammatory demyelinating polyneuropathy, Guillain-Barré Syndrome, central pontine myelinosis, Marchiafava-Bignami disease, Charcot Marie Tooth, leukodystrophy or schizophrenia.

Within this list of diseases, blast-induced mild traumatic brain injury is particularly preferred.

As discussed herein above, implicit memory tasks were used in the examples herein below on blasted mice displaying blast-induced mild traumatic brain injury to reverse the blast-induced FA changes, including myelin abnormalities.

In a preferred embodiment of the implicit memory task for the use as defined-above the implicit memory task is selected from the group consisting of walking, cycling, running, swimming, puzzling, juggling, balancing, mirror tracing, reading reversed text, doing a word-completion task, and singing a familiar song.

These non-limiting examples of implicit memory tasks should illustrate implicit memory tasks which can be used in accordance with the invention. These implicit memory tasks are in particular useful if the subject is human.

In a further preferred embodiment of the implicit memory task for the use as defined-above, the implicit memory task is delay fear conditioning or trace fear conditioning, wherein the delay fear conditioning comprises (i) a conditioning stimulus selected from the group consisting of an auditory stimulus, a visual stimulus, a taste stimulus and a smell, and (ii) an unconditioned stimulus selected from the group consisting of an electric stimulus, heat, pain and affrighting, and wherein the unconditioned stimulus is applied after the conditioning stimulus.

In accordance with this embodiment the subject may also be a non-human subject. The term "after" preferably means directly after. Directly after means, with increasing preference, within 1 min, 30sec, 15sec, 10sec, 5sec, 2sec, 1sec in the case of trace fear conditioning. Most preferably directly after means around 0 sec and hence without any time delay. In delay fear conditioning, the trace is around 0 sec.

As it can be taken from the examples, delay fear conditioning was used to reverse the blast-induced FA changes, including myelin abnormalities in blasted mice.

In a more preferred embodiment of the implicit memory task for the use as defined-above, wherein the subject is a mouse, the auditory stimulus is a sound and the electric stimulus is applied to a paw of the mouse.

These conditions match the implicit memory tasks used in the examples.

In a third embodiment the present invention relates to a method of monitoring the regeneration of myelin abnormalities in a subject *in vivo* by measuring fractional anisotropy in the brain of the subject by diffusion tensor imaging, wherein the regeneration of the myelin abnormalities is indicated by an increase of fractional anisotropy.

This method may particularly be useful for diagnostic purposes or to adjust the drug dosage regimen for a particular subject. Regression as well as progression of myelin abnormalities may be monitored. The definitions of terms in the context of the first and second embodiment of the invention apply mutatis mutandis to the third embodiment of the invention.

**Example 1 - Material and Methods**

**Shock tube**

A shock tube was used to deliver shock waves, similar to those seen in free field blasts, to the mice. When the Helium gas in the Driver Section of the shock tube reaches the maximal bursting pressure of the Mylar diaphragm, a pressure wave travels down the tube. The shock wave hits the head of the mouse which is located at the very end of the shock tube. The mouse is attached to a vertical board in the Mouse Chamber with the top of the head facing the shock tube. A pad is placed behind the mouse head to allow a maximum of 45 degree head rotation in order to prevent neck injury. The pressure at the tube exit was uniform across the entire exit plane as determined with a pressure gauge. 12, 16, and 19 psi were applied and found that exposure to 16 psi was linked to a mortality rate of 10%, whereas exposure to 19 psi was not survived by any C57BL/6N mouse. In this study, a Helium-driven pressure wave was selected that exhibited a constant overpressure of 16.02 +/- 0.60 psi for 1.4 msec. Helium was selected as the driver gas due to its low atomic weight and high speed of sound. Therefore, the pressure curve rises faster than if it was driven by air and becomes similar to the pressure curves from IEDs in the war zones. The duration of the positive phase of the shock wave was kept as short as possible to better scale to human exposure. Using Holbourn's scaling laws as a guide, time duration scaling is:

$$\frac{t_2}{t_1} = \left(\frac{m_1}{m_2}\right)^{-1/3}$$

It can be seen that the time duration scaling factor increases as the mass differences also increase (17). A 1.4 msec shock wave to a 480 mg mouse brain ($m_1$) would roughly scale to a 20 msec shock wave to a 1350 g human brain ($m_2$). While 20 msec is an extreme case for humans, long duration positive pressure shock waves can be experienced when exposed to large explosions at a distance. The software ConWep (18) predicts a positive phase blast duration of 14 msec from 136 kg of TNT at 15 m which is possible by a vehicle IED.

**Animal preparation**

Male C57BL/6N mice, 8 weeks old, were purchased from Harlan Laboratories and kept single-housed in the animal facility of Northwestern University for at least one week. Animals' care was in accordance with institutional guidelines of Northwestern University. All experimental procedures were approved by the Northwestern University Animal Care and Use Committee. On Day 1 of the experiment, the mice individually were anesthetized in a plastic transparent induction box (22.9 x 12 x 11.5 cm) with 4% isoflurane, and then protected the body except for the head with a ballistic Nylon coat. The animals' ears were protected with 1.5 x 1.5 mm foam plugs (pura-fit, Moldex-Metric INC, Culver City, California) placed into the ears. Isoflurane anesthesia (2%; 0.5 l / min) was maintained through flexible tubing to the nose of the mouse. Subsequently, the mice were blasted as described above. The animals needed an average of 7.1 min to recover in their own cages after the blast. Full recovery was indicated by the presence of a grasp reflex (19) and a coordinated walk on the top of an overturned cage cover. The recovery time of blasted mice was three times longer than the recovery time of the non-blasted mice which were treated like the blasted animals except for the missing blast. On Day 3, all mice were subjected to training for auditory trace or delay fear conditioning. On Day 4, all mice were tested for conditioned fear to the training context and then to the auditory cue in a novel context. On Day 14, all mice were subjected to cardiac perfusion. Subsequently, the isolated brains were analyzed with DTI in a 14.1 TESLA MR imager. Totally, five groups were used. Two of the five groups were blasted and then subjected to auditory trace fear conditioning (TFC) or auditory delay fear conditioning (DFC). Two additional groups served as controls that were TFC- or DFC-treated, but not blasted. A fifth group provided non-blasted, non-trained naïve controls.

**Fear Conditioning**

Separate groups of mice were used for TFC and DFC. The two paradigms were identical except that TFC included a 15 s stimulus free trace period between the end of the conditioning stimulus (CS) and the start of the unconditioned stimulus (US). The CS was a 30 s, 75 dB, broadband noise pulsed at 5 Hz with a 50% duty cycle and a 5 ms rise-fall time. The CS was presented 180 s after the mouse was placed onto a 25 x 29 cm grid floor in a 29 cm high conditioning chamber (Coulbourn) scented with Clidox cleaner/disinfectant. The US was a 2 s, 0.7 mA scrambled shock (DC) to the paws that was delivered at the end of the CS for DFC, or at the end of the trace interval for TFC. Mice were given one conditioning trial, remained in the conditioning chamber for approximately 30 seconds and were then returned to their home-cages. They were returned to the conditioning chamber for three minutes approximately 24 hours later to test for contextual fear, i.e. freezing to the conditioning chamber, and were then returned to their home-cage for approximately one hour. The mice were then placed in a novel 18 cm tall chamber covering an area of 34 x 28 $cm^2$. The chamber was made of plastic, had a smooth floor, was scented with Windex cleaner, and illuminated with red, instead of white light. A single 60 s CS was delivered after a 180 s baseline period, and data were recorded for another 180 s period after the CS. The experiment was done inside an Industrial Acoustics Corporation acoustical cabinet and was controlled by Actimetrics' FreezeFrame software.

**Diffusion Tensor Imaging**

*Ex vivo* DTI of the paraformaldehyde-fixed mouse brains were acquired with a multi-slice spin-echo diffusion weighted imaging sequence on a 14.1 Tesla Bruker vertical bore MR imager. The following parameters were used: TR= 3000ms, TE= 16ms, time between diffusion gradient pulses $\Delta$=7ms, duration of diffusion gradient $\delta$=3 ms, slice thickness=500$\mu$m, field of view=15mmx15mm, and matrix size= 256x256 yielding an in-plane voxel size of 59$\mu$m. Diffusion sensitizing gradients were applied along 30 directions with a single *b value of 1000* s/mm2. DTI data were processed using Bruker ParaVison software, and fractional anisotropy (FA) was calculated for medial and lateral corpus callosum from four slices spanning from near splenium to genu. The FA values were averaged across hemispheres and slices for each mouse.

Impact of DFC and TFC on FA in the medial corpus callosum after blast: Nonblasted mice subjected to DFC or TFC reached significantly greater FA than naïve mice (nonblasted, non-conditioned). After blast, this significant difference was maintained with the DFC-, but not with the TFC-treated mice. FA values between blasted and non-blasted TFC-treated mice differed significantly.

In the lateral corpus callosum: No significant FA differences were found when naïve, non-blasted or blasted DFC-treated, or non-blasted TFCtreated mice were compared. Only the blasted TFC-treated mice showed significantly lower FA values than the naïve mice, the blasted DFC-treated mice or the non-blasted TFC-treated mice.

Images used for DTI: Four slices were analyzed per mouse, representing corpus callosum near splenium (= Bregma, P 2.5 mm), posterior body (P 1.5 and P 0.5 mm), and near genu, anterior body (A 1.0 mm).

Correlation analysis of FA values and freezing. A Z test (StatView5.0) revealed a significant correlation of -0.603 (p=0.0004) between FA in the lateral corpus callosum and freezing to the training context.

**Cardiac perfusion**

Mice were anesthetized with a drug cocktail of ketamine (87 mg/kg) and xylazine (13 mg/kg) injected i.p. Depth of anesthesia was ascertained by absence of response to toe pinch. Anesthetized mice were pinned on a slanted board and the chest wall was opened by incision to expose the heart. A 25-gauge needle was inserted into the left ventricle, and an incision was made in the right atrium to drain blood. Heparinized saline was allowed to flow via the needle into the left ventricle. Perfusion of saline was continued for approximately 10-15 ml or until the return flow was colorless. Saline was then replaced with 4% paraformaldehyde, and perfusion was continued for another 10-15 ml. The skull was then opened, the brain removed and placed in 4% paraformaldehyde for further processing with DTI. Prior to imaging, brains were incubated in 0.2 M sodium phosphate buffer (pH 7.4) for 48 hours and transferred to sample holders filled with perfluoro polyester (Fomblin).

**Data Analysis**

Video data from fear conditioning sessions were analyzed with Actimetrics' FreezeView software. A threshold for movement was identified for each mouse based on a frequency distribution of the motion index (a measure of the number of pixels that changed their signal between adjacent video frames). A sharp inflection of the graph was typical and indicated the value that discriminated freezing from movement. A bout of at least 1 sec was required to be considered freezing (finer resolution created noisy data). Cued freezing was determined by comparing percent freezing during the 60 sec exposure to cue with the percent freezing during the 60 sec prior to cue onset. The data were also normalized to baseline levels of activity so that cue evoked freezing could be detected upon a background of increased freezing, i.e., if there was generalized fear to a novel context. Normalized freezing was calculated as: ((cue freezing - preCue freezing)/preCue freezing) x100. An Excel file of percent freezing was generated for each batch of files, and StatView was used to perform statistical analyses. FreezeView also generated a "fraster" (raster plot of freezing bouts for each mouse) to show the time-course of freezing for each mouse in a group. A 2 x 2 factorial ANOVA (blast/no blast x trace/delay fear conditioning) was used for the primary analysis. Fisher's PLSD tests were used for post-hoc analyses. FA data were averaged for each group of mice and analyzed with t test (two tailed equal variant).

**Example 2 - *Ex vivo* DTI after blast and auditory TFC or DFC**

FA analysis revealed that subjecting non-blasted mice to auditory TFC or DFC significantly increased FA in the medial part of the corpus callosum in comparison to naïve mice that were not exposed to blast or any behavioral paradigm . In the lateral corpus callosum, naïve and non-blasted DFC- or TFC-treated mice did not significantly differ in FA . However, the FA values of mice that were blasted and subsequently subjected to TFC were significantly reduced as compared to the FA values of non-blasted TFC-treated mice. In contrast, when mice were treated with DFC after blast, the FA values were not significantly different from those found in the non-blasted DFC-treated mice. It was observed earlier on Day 4 that blasting the mice did not produce significant freezing differences between mice subjected to auditory TFC or auditory DFC. In contrast, after Day 14 it was observed that mice which had received a blast and had been subjected to auditory TFC showed significantly lower FA values than blasted mice that had been subjected to auditory DFC.

**Example 3- Correlation between fractional anisotropy and freezing**

Statistical significance of the correlation between FA and freezing behavior was determined using a Z test (StatView 5.0). All four groups were combined for this analysis to determine whether FA values were related to freezing. Correlation between FA and contextual freezing was significant in the lateral corpus callosum and in the medial corpus callosum. Normalized cued freezing was significantly correlated with FA in the medial corpus callosum (r=0.489; p=0.028), but not in the lateral corpus callosum.

**References:**

1. J. A. Langlois, W. Rutland-Brown, The Epidemiology and Impact of Traumatic Brain Injury: A Brief Overview. J Head Trauma Rehabil. 21: 375-378 (2006).

2. R. A. Shults, B.H. Jones, M. J. Kresnow, J. A. Langlois, J. L. Guerrero, Disability among adults injured in motor vehicle crashes in the United States. J. Safety Research. 35: 447-452 (2004).

3. G. Ling, F. Bandak, R. Armonda, G. Grant, and J. Ecklund, Explosive Blast Neurotrauma. Journal of Neurotrauma 26: 815-825 (2009).

4. G. Ling, and J. Ecklund, Traumatic brain injury in modern war. Curr Opin Anesthesiol 24:124-130 (2011).

5. L. D. Landau and E. M. Lifshitz, Shock Waves. Fluid Mechanics 6: 310-346 (1969).

6. W. E. Baker, Blast Pressure Effects: An Overview. In R. Scott (Ed), In: Design Considerations for Toxic Chemical and Explosives Facilities. American Chemical Society: pp2-57 (1987). Washington, DC.

7. J. H. Stuhmiller, Y. Y. Phillips, and D. R. Richmond, (1991) The Physics and Mechanisms of Primary Blast Injury. In: Conventional Warfare, Ballistic, Blast and Burn Injuries (Part I. Warfare, Weaponry, and the Casualty) (R. F. Bellamy, and R. Zajtchuk, eds.) Office of the Surgeon General at TMM Publications.pp.241-270 (1991). Washington, DC

8. R. C. Hall, R. C. Hall, M. J. Chapman, Definition, Diagnosis, and Forensic Implications of Postconcussional Syndrome. Psychosomatics 46: 195-202 (2005).

9. S. N. Niogi, P. Mukherjee, J. Ghajar, C. Johnson, R. A. Kolster, R. Sarkar, H. Lee, M. Meeker, R. D. Zimmerman, G. T. Manley, B. D. McCandlis, Extent of microstructural white matter injury in postconcussive syndrome correlates with impaired cognitive reaction time: a 3T diffusion tensor imaging study of mild traumatic brain injury. Am J Neuroradiol. 29: 967-73 (2008).

10. DoD Numbers for Traumatic Brain Injury, Worldwide - Totals, http://www.health.mil/Libraries/TBI-Numbers-Current-Reports/dod-tbi-worldwide-2000-2012Q1-as-of-120516.pdf [accessed 5 July 2012].

11. C. L. MacDonald, K. Dikranian, S. K. Song, P. V. Bayly, D. M. Holtzman, D. L. Brody, Detection of traumatic axonal injury with diffusion tensor imaging in a mouse model of traumatic brain injury, Experimental Neurology 205: 116-131 (2007).

12. C. W. Hoge CW, McGurk D, Thomas JL, Cox AL, Engel CC, Castro CA, Mild traumatic brain injury in U.S. Soldiers returning from Iraq. N Engl J Med. 358: 453-63 (2008).

13. D. H. Smith, and D. F. Meaney, Axonal damage in traumatic brain injury. The Neuroscientist 6, 483-495 (2000).

14. L. R. Squire, S. M. Zola, Structure and function of declarative and nondeclarative memory systems. Proc. Natl. Acad. Sci. USA 93: 13515-13522 (1996).

15. R. E. Clark, J. R. Manns, L. R. Squire, Classical conditioning, awareness, and brain systems. Trends Cogn Sci 6: 524-531 (2002).

16. J. Spiess, P. H. Rigby, P. Tovote, U. Schnitzbauer, T. Ji, A. M. Wyrwicz, J Neurotrauma 27, 5, A-14 (2010).

17. A. K. Ommaya, P. Yarnell, A. E. Hirsch, and E. H. Harris, Scaling of Experimental Data on Cerebral Concussion in Sub-Human Primates to Concussion Threshold for Man. 11th Stapp Car Crash Conference, Anaheim, California, SAE International Warrendale, Pennsylvania, USA (1967).

18. D. W. Hyde, ConWep 2.1.0.8, Conventional Weapons Effects Program (United States Army Corps of Engineers, Vicksburg, MS), (2004).

19. W. M. Fox, Reflex-ontogeny and behavioural development of the mouse. Anim Behav. 13:234-41 (1965).

20. V. Rubovitch, M. Ten-Bosch, O. Zohar, C. R. Harrison, C. Tempel-Brami, E. Stein, B. J. Hoffer, C. D. Balaban, S. Schreiber, W. T. Chiu, C. G. Pick, A mouse model of blast-induced mild traumatic brain injury. Experimental Neurology 232:280-289 (2011).

21. I. Cernak, and L. J. Noble-Haeusslein, Traumatic brain injury: an overview of pathobiology with emphasis on military populations. Journal of Cerebral Blood Flow & Metabolism 30: 255-266 (2010).

22. M. Risling, S. Plantman, M. Angeria, E. Rostami, B.M. Bellander, M. Kirkegaard, U. Arborelius, J. Davidsson, Mechanisms of blast induced brain injuries, experimental studies in rats. Neuroimage 54 Suppl 1: S89-97 (2011).

23. V. E. Koliatsos, I. Cernak, L. Xu, Y. Song, A. Savonenko, B. J. Crain, C. G. Eberhart, C. E. Frangakis, T. Melnikova, H. Kim, D. Lee, (2011) A mouse model of blast injury to brain: initial pathological, neuropathological, and behavioral characterization. J Neuropathol Exp Neurol 70;399-416 (2011).

24. R. Bolander, B. Mathie, C. Bir, D. Ritzel, and P. VandeVord, Skull flexure as a contributing factor in the mechanism of injury in the rat when exposed to shock waves. Annals of Biomedical engineering 39: 2550-2559 (2011).

25. L. E. Goldstein, A. M. Fisher, C. A. Tagge, X. L. Zhang, L. Velisek, J. A. Sullivan, C. Upreti, J. M. Kracht, M. Ericsson, M. W. Wojnarowicz, C. J. Goletiani, G. M. Maglakelidze, N. Casey, J. A. Moncaster, O. Minaeva, R. D. Moir, C. J. Nowinski, R. A. Stern, R. C. Cantu, J. Geiling, J. K. Blusztajn, B. L. Wolozin, T. Ikezu, T. D. Stein, A. E. Budson, N. W. Kowall, D. Chargin, A. Sharon, S. Saman, G. F. Hall, W. C. Moss, R. O. Cleveland, R. E. Tanzi, P. K. Stanton, A. C. McKee, Chronic traumatic encephalopathy in blast-exposed military veterans and a Blast Neurotrauma Mouse Model. Science Translational Medicine 4, 1-16 (2012).

26. N. Kaouane, Y. Porte, M. Vallee, L. Brayda-Bruno, N. Mons, L. Calanndreau, A. Marighetto, P. V.

Piazza, A. Desmedt, Glucocorticoids can induce PTSD-like memory impairments in mice. Sciencexpress 23. February 2012, pages 1-5/10.1126/science (2012).1207615.

27. S. W. Sun, J. J. Neil, H. F. Liang, Y. Y. He, R. E. Schmidt, C. Y. Hsu, S. K. Song, (2005) Formalin fixation alters water diffusion coefficient magnitude but not anisotropy in infarcted brain. Magn Reson Med., 53:1447-51 (2005).

28. H. E. D'Arceuil, S. Westmoreland, A. J. de Crespigny, An approach to high resolution diffusion tensor imaging in fixed primate brain. Neuroimage,35: 553-65, (2007).

29. R. J. Zatorre, R. D. Fields, H. Hohansen-Berg, Plasticity in gray and white: neuroimaging changes in brain structure during learning. Nature Neuroscience 15, 528-536 (2012).

30. H. Takeuchi, A. Sekiguchi, Y. Taki, S. Yokoyama, Y. Yomogida, N. Komuro, T. Yamanouchi, S. Suzuki, and R. Kawashima, Training of working memory impacts structural connectivity. Journal of Neuroscience 30:3297-3303 (2010).

31. Jan Scholz, Miriam C. Klein,Timothy E.J. Behrens, and Heidi Johansen-Berg, Training induces changes in white-matter architecture. Nature Neuroscience, published online 11 October 2009; doi: 10.1038/nn.2412

**Claims**

1. A method of screening for a compound decreasing axonal injury, comprising the steps of

    (a) exposing the head of a non-human mammal to a pressure of 12 to 17 psi in a shock tube for a time $t_2$ calculated by the formula

    $$t_2 = (m_1/m_2)^{-1/3} \times t_1,$$

    wherein $m_2$ is the weight of the brain of the non-human mammal, $m_1$ is 480 mg and $t_1$ is 1.0 to 2.0 msec,
    (b) administering a test compound to the non-human mammal obtained in (a), and/or
    (b') administering a test compound to the non-human mammal before step (a), and
    (c) measuring the fractional anisotropy in the brain of the non-human mammal by diffusion tensor imaging after performing steps (a) and (b), or (a) and (b'), or (a), (b) and (b'), wherein a test compound is a compound decreasing axonal injury if

    (i) in the case of (b) and/or (b') the fractional anisotropy measured in step (c) is increased as compared to the fractional anisotropy measured in the brain of a control non-human mammal of the same species which has been exposed to pressure as defined in step (a), and/or
(ii) in the case of (b) the fractional anisotropy measured in step (c) is increased as compared to the fractional anisotropy measured in the brain of said non-human mammal after step (a) and before the administration of the test compound in step (b).

2. The method according to claim 1, wherein the non-human mammal is a mouse.

3. The method of claim 2 or 3, wherein the shock tube generates a helium-driven pressure wave.

4. The method according to any one of claims 1 to 3, wherein step (c) is performed *ex vivo* or *in vivo.*

5. The method according to any one of claims 1 to 4, wherein measuring the fractional anisotropy in the brain comprises measuring the fractional anisotropy in the white matter.

6. The method according to claim 5, wherein measuring the fractional anisotropy in the white matter comprises measuring the fractional anisotropy in the corpus callosum.

7. The method according to any one of claims 2 to 6, wherein the head of the mouse is exposed to 16.02 ± 0.6 psi for a time of 1.4 msec.

8. The method according to any one of claims 1 to 7, wherein the control non-human mammal of the same species is of the same race.

9. An implicit memory task for use in the treatment of myelin abnormalities in a subject.

10. The implicit memory task for the use according to claim 9, wherein the myelin abnormalities are demyelination and/or dysmyelination.

11. The implicit memory task for the use according to claim 9 or 10, wherein the subject has a traumatic brain injury, blast-induced mild traumatic brain injury, multiple sclerosis, neuromyelitis optica, acute disseminated encephalomyelitis, transverse myelitis, chronic inflammatory demyelinating polyneuropathy, Guillain-Barré Syndrome, central pontine myelinosis, Marchiafava-Bignami disease, Charcot Marie Tooth, leukodystrophy or schizophrenia.

12. The implicit memory task for the use according to any one of claims 9 to 11, wherein the implicit memory task is selected from the group consisting of walking, cycling, running, swimming, puzzling, juggling, balancing, mirror tracing, reading reversed text, doing a word-completion task, and singing a familiar song.

13. The implicit memory task for the use according to any one of claims 9 to 11, wherein the implicit memory task is delay fear conditioning, wherein the delay fear conditioning comprises

(i) a conditioning stimulus selected from the group consisting of an auditory stimulus, a visual stimulus, a taste stimulus, and a smell; and
(ii) an unconditioned stimulus selected from the group consisting of an electric stimulus, heat, pain, and affrighting,
and wherein the unconditioned stimulus is applied after the conditioning stimulus.

14. The implicit memory task for the use according to claim 13, wherein the subject is a mouse, the auditory stimulus is a sound and the electric stimulus is applied to a paw of the mouse.

15. A method of monitoring the regeneration of myelin abnormalities in a subject *in vivo* by measuring fractional anisotropy in the brain of the subject by diffusion tensor imaging, wherein the regeneration of the myelin abnormalities is indicated by an increase of fractional anisotropy.

Europäisches
Patentamt

European
Patent Office

Office européen
des brevets

# EUROPEAN SEARCH REPORT

Application Number

EP 12 18 0656

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | VARDIT RUBOVITCH ET AL: "A mouse model of blast-induced mild traumatic brain injury", EXPERIMENTAL NEUROLOGY, ACADEMIC PRESS, NEW YORK, NY, US, vol. 232, no. 2, 9 September 2011 (2011-09-09), pages 280-289, XP028325411, ISSN: 0014-4886, DOI: 10.1016/J.EXPNEUROL.2011.09.018 [retrieved on 2011-09-17] * page 281, left-hand column, line 22 - page 282, left-hand column, line 45 * * page 282, right-hand column, line 51 - page 284, right-hand column, line 12 * * page 285, right-hand column, line 23 - page 286, left-hand column, line 10 * * page 287, left-hand column, line 11 - page 288, left-hand column, line 10 * * figure 1 * ----- | 1,2,4-14 | INV. A61B5/055 |
| A | CERNAK I ET AL: "The pathobiology of blast injuries and blast-induced neurotrauma as identified using a new experimental model of injury in mice", NEUROBIOLOGY OF DISEASE, BLACKWELL SCIENTIFIC PUBLICATIONS, OXFORD, GB, vol. 41, no. 2, 1 February 2011 (2011-02-01), pages 538-551, XP027580068, ISSN: 0969-9961 [retrieved on 2010-12-30] * page 539, left-hand column, line 22 - right-hand column, line 23 * * page 540, line 1, paragraph r - page 541, line 41, paragraph r * * figure 1; table 3 * ----- -/-- | 1-3,7-14 | TECHNICAL FIELDS SEARCHED (IPC) A61B G01R |

The present search report has been drawn up for all claims

| Place of search | Date of completion of the search | Examiner |
|---|---|---|
| The Hague | 19 March 2013 | Chen, Amy |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 12 18 0656

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LAURA A. HARSAN ET AL: "Brain dysmyelination and recovery assessment by noninvasive in vivo diffusion tensor magnetic resonance imaging", JOURNAL OF NEUROSCIENCE RESEARCH, vol. 83, no. 3, 15 February 2006 (2006-02-15), pages 392-402, XP055056561, ISSN: 0360-4012, DOI: 10.1002/jnr.20742 | 15 | |
| A | * page 393, right-hand column, line 26 - page 394, left-hand column, line 11 * <br> * page 395, line 14, paragraph r - page 396, line 7, paragraph r * <br> * page 399, left-hand column, line 8 - page 401, left-hand column, line 26 * <br> * page 399, left-hand column, line 9 - page 401, left-hand column, line 26 * <br> * figure 1; table 1 * <br> ----- | 1,4-6,8 | |
| A | US 6 529 763 B1 (COHEN YORAM [IL] ET AL) 4 March 2003 (2003-03-04) <br> * column 3, line 35 - column 4, line 65 * <br> * column 9, line 60 - column 10, line 39 * <br> ----- | 1,5,6,15 | |

TECHNICAL FIELDS SEARCHED (IPC)

The present search report has been drawn up for all claims

| Place of search | Date of completion of the search | Examiner |
|---|---|---|
| The Hague | 19 March 2013 | Chen, Amy |

EPO FORM 1503 03.82 (P04C01)

**ANNEX TO THE EUROPEAN SEARCH REPORT
ON EUROPEAN PATENT APPLICATION NO.**   EP 12 18 0656

This annex lists the patent family members relating to the patent documents cited in the above-mentioned European search report.
The members are as contained in the European Patent Office EDP file on
The European Patent Office is in no way liable for these particulars which are merely given for the purpose of information.

19-03-2013

| Patent document cited in search report | Publication date | Patent family member(s) | Publication date |
|---|---|---|---|
| US 6529763 B1 | 04-03-2003 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4946778 A
- US 6080560 A

### Non-patent literature cited in the description

- Design of Prodrugs. Elsevier, 1985
- **HARLOW ; LANE.** Antibodies, A Laboratory Manual. Cold Spring Harbor Laboratory Press, 1988
- **HARLOW ; LANE.** Using Antibodies: A Laboratory Manual. Cold Spring Harbor Laboratory Press, 1999
- **KÖHLER ; MILSTEIN.** *Nature,* 1975, vol. 256, 495-497
- **KOZBOR.** *Immunology Today,* 1983, vol. 4, 72
- **COLE et al.** Monoclonal Antibodies and Cancer Therapy. Alan R. Liss, Inc, 1985, 77-96
- **SCHIER.** *Human Antibodies Hybridomas,* 1996, vol. 7, 97-105
- **MALMBORG.** *J. Immunol. Methods,* 1995, vol. 183, 7-13
- **OSBORNE.** *Current Opinion in Chemical Biology,* 1997, vol. 1, 5-9
- **STULL ; SZOKA.** *Pharmaceutical Research,* 1995, vol. 12 (4), 465-483
- **FOUGEROLLES et al.** *Current Opinion in Pharmacology,* 2008, vol. 8, 280-285
- **LU et al.** Methods in Molecular Biology, vol. 437: Drug Delivery Systems. 2008, vol. 437
- **MELANI et al.** *Cancer Res.,* 1991, vol. 51, 2897-2901
- **KUBINYI.** Hausch-Analysis and Related Approaches. VCH Verlag, 1992
- **HOLZGRABE ; BECHTOLD.** *Deutsche Apotheker Zeitung,* 2000, vol. 140 (8), 813-823
- **LE BIHAN, D ; E. BRETON.** *C R Acad Sci (Paris,* 1985, vol. 301 (15), 1109-1112
- **HAGMANN et al.** *Radio Graphics,* October 2006
- **ISMRM '97, VANCOUVER CANADA.** *ISMRM '97, Vancouver Canada,* 1997, 1742
- **WESTIN et al.** Processing and visualization of diffusion tensor MRI. *Medical Image Analysis,* 2002, vol. 6 (2), 93-108
- **HAGMANN et al.** *RadioGraphics,* October 2006

- **J. A. LANGLOIS ; W. RUTLAND-BROWN.** The Epidemiology and Impact of Traumatic Brain Injury: A Brief Overview. *J Head Trauma Rehabil,* 2006, vol. 21, 375-378
- **R. A. SHULTS ; B.H. JONES ; M. J. KRESNOW ; J. A. LANGLOIS ; J. L. GUERRERO.** Disability among adults injured in motor vehicle crashes in the United States. *J. Safety Research.,* 2004, vol. 35, 447-452
- **G. LING ; F. BANDAK ; R. ARMONDA ; G. GRANT ; J. ECKLUND.** Explosive Blast Neurotrauma. *Journal of Neurotrauma,* 2009, vol. 26, 815-825
- **G. LING ; J. ECKLUND.** Traumatic brain injury in modern war. *Curr Opin Anesthesiol,* 2011, vol. 24, 124-130
- **L. D. LANDAU ; E. M. LIFSHITZ.** Shock Waves. *Fluid Mechanics,* 1969, vol. 6, 310-346
- Blast Pressure Effects: An Overview. **W. E. BAKER.** Design Considerations for Toxic Chemical and Explosives Facilities. American Chemical Society, 1987, 2-57
- The Physics and Mechanisms of Primary Blast Injury. **J. H. STUHMILLER ; Y. Y. PHILLIPS ; D. R. RICHMOND.** Conventional Warfare, Ballistic, Blast and Burn Injuries. Office of the Surgeon General at TMM Publications, 1991, 241-270
- **R. C. HALL ; R. C. HALL ; M. J. CHAPMAN.** Definition, Diagnosis, and Forensic Implications of Postconcussional Syndrome. *Psychosomatics,* 2005, vol. 46, 195-202
- **S. N. NIOGI ; P. MUKHERJEE ; J. GHAJAR ; C. JOHNSON ; R. A. KOLSTER ; R. SARKAR ; H. LEE ; M. MEEKER ; R. D. ZIMMERMAN ; G. T. MANLEY.** Extent of microstructural white matter injury in postconcussive syndrome correlates with impaired cognitive reaction time: a 3T diffusion tensor imaging study of mild traumatic brain injury. *Am J Neuroradiol,* 2008, vol. 29, 967-73
- *DoD Numbers for Traumatic Brain Injury, Worldwide - Totals,* 05 July 2012, http://www.health.mil/Libraries/TBI-Numbers-Current-Reports/dod-tbi-worldwide-2000-2012Q1-as-of-120516.pdf

- **C. L. MACDONALD ; K. DIKRANIAN ; S. K. SONG ; P. V. BAYLY ; D. M. HOLTZMAN ; D. L. BRODY.** Detection of traumatic axonal injury with diffusion tensor imaging in a mouse model of traumatic brain injury. *Experimental Neurology,* 2007, vol. 205, 116-131
- **C. W. HOGE CW ; MCGURK D ; THOMAS JL ; COX AL ; ENGEL CC ; CASTRO CA.** Mild traumatic brain injury in U.S. Soldiers returning from Iraq. *N Engl J Med,* 2008, vol. 358, 453-63
- **D. H. SMITH ; D. F. MEANEY.** Axonal damage in traumatic brain injury. *The Neuroscientist,* 2000, vol. 6, 483-495
- **L. R. SQUIRE ; S. M. ZOLA.** Structure and function of declarative and nondeclarative memory systems. *Proc. Natl. Acad. Sci. USA,* 1996, vol. 93, 13515-13522
- **R. E. CLARK ; J. R. MANNS ; L. R. SQUIRE.** Classical conditioning, awareness, and brain systems. *Trends Cogn Sci,* 2002, vol. 6, 524-531
- **J. SPIESS ; P. H. RIGBY ; P. TOVOTE ; U. SCHNITZBAUER ; T. JI ; A. M. WYRWICZ.** *J Neurotrauma,* 2010, vol. 27 (5), A-14
- **A. K. OMMAYA ; P. YARNELL ; A. E. HIRSCH ; E. H. HARRIS.** *Scaling of Experimental Data on Cerebral Concussion in Sub-Human Primates to Concussion Threshold for Man. 11th Stapp Car Crash Conference, Anaheim, California, SAE International Warrendale, Pennsylvania, USA,* 1967
- **D. W. HYDE.** *ConWep 2.1.0.8, Conventional Weapons Effects Program (United States Army Corps of Engineers, Vicksburg, MS,* 2004
- **W. M. FOX.** Reflex-ontogeny and behavioural development of the mouse. *Anim Behav,* 1965, vol. 13, 234-41
- **V. RUBOVITCH ; M. TEN-BOSCH ; O. ZOHAR ; C. R. HARRISON ; C. TEMPEL-BRAMI ; E. STEIN ; B. J. HOFFER ; C. D. BALABAN ; S. SCHREIBER ; W. T. CHIU.** A mouse model of blast-induced mild traumatic brain injury. *Experimental Neurology,* 2011, vol. 232, 280-289
- **I. CERNAK ; L. J. NOBLE-HAEUSSLEIN.** Traumatic brain injury: an overview of pathobiology with emphasis on military populations. *Journal of Cerebral Blood Flow & Metabolism,* 2010, vol. 30, 255-266
- **M. RISLING ; S. PLANTMAN ; M. ANGERIA ; E. ROSTAMI ; B.M. BELLANDER ; M. KIRKEGAARD ; U. ARBORELIUS ; J. DAVIDSSON.** Mechanisms of blast induced brain injuries, experimental studies in rats. *Neuroimage,* 2011, vol. 54 (1), 89-97
- **V. E. KOLIATSOS ; I. CERNAK ; L. XU ; Y. SONG ; A. SAVONENKO ; B. J. CRAIN ; C. G. EBERHART ; C. E. FRANGAKIS ; T. MELNIKOVA ; H. KIM.** A mouse model of blast injury to brain: initial pathological, neuropathological, and behavioral characterization. *J Neuropathol Exp Neurol,* 2011, vol. 70, 399-416
- **R. BOLANDER ; B. MATHIE ; C. BIR ; D. RITZEL ; P. VANDEVORD.** Skull flexure as a contributing factor in the mechanism of injury in the rat when exposed to shock waves. *Annals of Biomedical engineering,* 2011, vol. 39, 2550-2559
- **L. E. GOLDSTEIN ; A. M. FISHER ; C. A. TAGGE ; X. L. ZHANG ; L. VELISEK ; J. A. SULLIVAN ; C. UPRETI ; J. M. KRACHT ; M. ERICSSON ; M. W. WOJNAROWICZ.** Chronic traumatic encephalopathy in blast-exposed military veterans and a Blast Neurotrauma Mouse Model. *Science Translational Medicine,* 2012, vol. 4, 1-16
- **N. KAOUANE ; Y. PORTE ; M. VALLEE ; L. BRAYDA-BRUNO ; N. MONS ; L. CALANNDREAU ; A. MARIGHETTO ; P. V. PIAZZA ; A. DESMEDT.** Glucocorticoids can induce PTSD-like memory impairments in mice. *Sciencexpress,* 23 February 2012, 1-510.1126
- *science,* 2012, 1207615
- **S. W. SUN ; J. J. NEIL ; H. F. LIANG ; Y. Y. HE ; R. E. SCHMIDT ; C. Y. HSU ; S. K. SONG.** Formalin fixation alters water diffusion coefficient magnitude but not anisotropy in infarcted brain. *Magn Reson Med.,* vol. 53, 1447-51
- **H. E. D'ARCEUIL ; S. WESTMORELAND ; A. J. DE CRESPIGNY.** An approach to high resolution diffusion tensor imaging in fixed primate brain. *Neuroimage,* 2007, vol. 35, 553-65
- **R. J. ZATORRE ; R. D. FIELDS ; H. HOHANSEN-BERG.** Plasticity in gray and white: neuroimaging changes in brain structure during learning. *Nature Neuroscience,* 2012, vol. 15, 528-536
- **H. TAKEUCHI ; A. SEKIGUCHI ; Y. TAKI ; S. YOKOYAMA ; Y. YOMOGIDA ; N. KOMURO ; T. YAMANOUCHI ; S. SUZUKI ; R. KAWASHIMA.** Training of working memory impacts structural connectivity. *Journal of Neuroscience,* 2010, vol. 30, 3297-3303
- **JAN SCHOLZ ; MIRIAM C. KLEIN ; TIMOTHY E.J. BEHRENS ; HEIDI JOHANSEN-BERG.** Training induces changes in white-matter architecture. *Nature Neuroscience,* 11 October 2009